(19) Europäisches Patentamt
European Patent Office
Office européen des brevets

(11) Veröffentlichungsnummer : **0 055 430**
**B1**

(12) # EUROPÄISCHE PATENTSCHRIFT

(45) Veröffentlichungstag der Patentschrift : **26.10.83**

(51) Int. Cl.³ : **C 07 C145/02**// A01N47/34

(21) Anmeldenummer : **81110569.1**

(22) Anmeldetag : **18.12.81**

(54) **N-(Sulfenamido)-acylisocyanate, Verfahren zu ihrer Herstellung und ihre Verwendung als Zwischenprodukte.**

(30) Priorität : **30.12.80 DE 3049448**

(43) Veröffentlichungstag der Anmeldung : **07.07.82 Patentblatt 82/27**

(45) Bekanntmachung des Hinweises auf die Patenterteilung : **26.10.83 Patentblatt 83/43**

(84) Benannte Vertragsstaaten : **AT BE CH DE FR GB IT LI NL**

(56) Entgegenhaltungen :
**EP A 0 007 439**

**CHEMISCHE BERICHTE, Jahrgang 99, Nr. 10, Seiten 3103-3107 A. HAAS et al. : « Beiträge zur Chemie des Trifluor methylsulfenyl Isocyanats und Bis trifluormethylsulfenyl uretidindions »**

(73) Patentinhaber : **BAYER AG**
**Zentralbereich Patente, Marken und Lizenzen**
**D-5090 Leverkusen 1, Bayerwerk (DE)**

(72) Erfinder : **Kühle, Engelbert, Dr.**
**von Bodelschwingh-Strasse 42**
**D-5060 Bergisch-Gladbach 2 (DE)**
Erfinder : **Hagemann, Hermann, Dr.**
**Kandinsky-Strasse 25**
**D-5090 Leverkusen 1 (DE)**

**EP 0 055 430 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

N-(Sulfenamido)-acylisocyanate, Verfahren zu ihrer Herstellung und ihre Verwendung als Zwischenprodukte

Die vorliegende Erfindung betrifft neue N-(Sulfenamido)-acylisocyanate, ein Verfahren zu ihrer Herstellung sowie ihre Verwendung als Zwischenprodukte für die Synthese von Fungiziden.

Es wurden neue N-(Sulfenamido)-acylisocyanate der allgemeinen Formel

$$R^1-S-\underset{R^2}{\underset{|}{N}}-CO-NCO \qquad (I)$$

in welcher

$R^1$ für einen Trihalogenmethyl-Rest steht und

$R^2$ für einen aliphatischen, cycloaliphatischen, araliphatischen, aromatischen oder heterocyclischen Rest steht,

gefunden.

Weiterhin wurde gefunden, daß man die Verbindungen der Formel I erhält, wenn man Trihalogenmethansulfenamide der allgemeinen Formel

$$R^1-S-\underset{R^2}{\underset{|}{NH}} \qquad (II)$$

in welcher

$R^1$ und $R_2$ die oben angegebene Bedeutung besitzen,

mit Chlorcarbonylisocyanat der Formel

$$Cl—CO—NCO \qquad (III)$$

gegebenenfalls in Gegenwart eines Verdünnungsmittels im Temperaturbereich zwischen 0 und 150 °C umsetzt.

Die neuen Verbindungen der Formel I sind interessante Zwischenprodukte zur Herstellung von Pflanzenschutzmitteln mit fungizider Wirkung.

Es ist überraschend, daß die erfindungsgemäße Umsetzung, bei der Chlorwasserstoff abgespalten wird, abläuft. Es ist nämlich bekannt, daß Sulfenamide leicht mit Chlorwasserstoff zu Sulfenylchloriden gespalten werden (vgl. Chem. Ber. *57*, 755 (1924) ; J. Gen. Chem. USSR (engl. transl.) *29*, 2129 (1959)). Nach dem Stand der Technik wäre deshalb zu erwarten gewesen, daß die bei höherer Temperatur erfolgende Chlorwasserstoff-Entwicklung eine Abspaltung des Sulfenylrestes bewirkt.

Von den erfindungsgemäßen N-(Sulfenamido)-acylisocyanaten der allgemeinen Formel I sind bevorzugt diejenigen, in denen

$R^1$ für einen Trichlormethyl-, Fluordichlormethyl-, Difluorchlormethyl- oder Trifluormethyl-Rest steht, und

$R^2$ für einen geradkettigen oder verzweigten Alkylrest mit 1 bis 18 Kohlenstoffatomen steht, der gegebenenfalls durch Alkoxi- und Alkylmercapto-Gruppen mit jeweils bis zu 4 Kohlenstoffatomen, ferner durch Halogen, Cyano- und Nitro-Gruppen substituiert sein kann, sodenn für einen Alkenyl- oder Alkinyl-Rest mit jeweils 3 bis 8 Kohlenstoffatomen, und für einen cycloaliphatischen Rest mit 5 bis 8 Kohlenstoffatomen steht, wobei letzterer gegebenenfalls durch einen Alkylrest mit 1 bis 4 Kohlenstoffatomen substituiert sein kann, ferner für einen araliphatischen Rest mit insgesamt 7 bis 12 Kohlenstoffatomen steht, dessen aromatisches Ringsystem gegebenenfalls durch Halogen, Nitro-, Trifluormethyl-, Cyano-, Alkyl- und Alkoxy-Gruppen mit jeweils bis zu 4 Kohlenstoffatomen substituiert sein kann, weiterhin noch für einen aromatischen Rest mit 6 bis 12 Kohlenstoffatomen steht, der gegebenenfalls durch Halogen, Nitro-, Halogenalkyl-, Alkyl-, Alkoxy-, Halogenalkoxy-, Alkylthio- und Halogenalkylthio mit jeweils bis zu 4 Kohlenstoff-atomen und Cyano-Gruppen substituiert sein kann, und endlich für einen heterocyclischen Rest mit 5 bis 6 Ringatomen steht, wobei 1 bis 3 Heteroatome wie Sauerstoff Schwefel und/oder Stickstoff im Ringsystem vorhanden sein können.

Ganz besonders bevorzugt sind diejenigen Bedeutungen der Substituenten, bei denen

$R^1$ für einen Trichlormethyl- oder Fluordichlormethyl-Rest steht und

$R^2$ für Alkyl mit bis zu 6 Kohlenstoffatomen steht, welches substituiert sein kann durch Halogen, Methoxy-, Ethoxy- und Methylthio-Gruppen, ferner für Cyclohexyl und für Phenyl steht, welches substituiert sein kann durch Halogen, Nitro-, Methyl-, Trifluormethyl- und Methoxy-Gruppen.

Verwendet man zur Herstellung einer erfindungsgemäßen Verbindung der Formel I beispielsweise Fluordichlormethansulfenyl-N-methylamid und Chlorcarbonylisocyanat als Ausgangsstoffe, so kann der Reaktionsverlauf durch das folgende Reaktionsschema wiedergegeben werden :

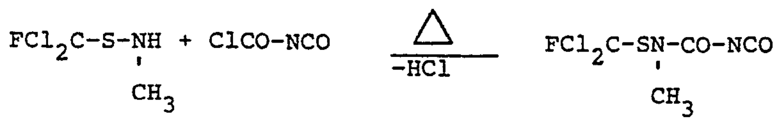

Die als Ausgangsmaterialien zu verwendenden Trihalogen-methan-sulfenamide sind durch die Formel II allgemein definiert ; in dieser Formel haben die Reste $R^1$ und $R^2$ vorzugsweise diejenigen Bedeutungen, die bereits im Zusammenhang mit der Beschreibung der erfindungsgemäßen Verbindungen der Formel I für diese Reste vorzugsweise genannt worden sind.

Die erfindungsgemäß verwendbaren Verbindungen der allgemeinen Formel II sind bekannt oder können nach bekannten Verfahren hergestellt werden (vgl. FR-PS 1 339 765 bzw. Chem. Abstr. *60,* 5519 (1964)). Man erhält sie, wenn man ein Trihalogenmethansulfenchlorid mit einem primären Amin beispielsweise in Toluol als Lösungsmittel im Temperaturbereich zwischen + 20 und 30 °C zur Umsetzung bringt (siehe auch die Herstellungsbeispiele).

Als Beispiele für Verbindungen der Formel (II) sind zu nennen die Trichlormethan-, Fluordichlormethan-, Difluorchlormethan- oder Trifluormethan-sulfenamide des Methyl-, Isopropyl-, Butyl-, Isooctyl-, Stearyl-, Allyl-, Propargyl-, Methoxyethyl-, Ethylmercaptoethyl-, β, β, β-Trifluorethyl-, Cyclopentyl-, 2-Methylcyclo-hexyl-, Benzyl-, 3-Nitro-benzyl- und Phenethyl-amins, sowie des Anilins, 4-Chloranilins, 3-Methoxyanilins, 3-Trifluormethylanilins, 1-Naphthylamins, 2-Furylamins und 4-Pyridylamins.

Das Chlorcarbonylisocyanat der Formel III ist aus der Literatur bekannt (vgl. Angew. Chem. *89,* 789 (1977)) und ausführlich beschrieben. Es wird zunächst das Additionsprodukt aus Phosgen und Chlorcyan am Aktivkohle-Kontakt hergestellt und das Addukt hernach mit Methansulfonsäure verseift, wobei man Chlorcarbonylisocyanat in 90 %iger Ausbeute erhält.

Die erfindungsgemäße Reaktion wird vorzugsweise in Anwesenheit eines Verdünnungsmittels durchgeführt. Als Verdünnungsmittel können alle inerten Lösungsmittel verwendet werden wie Kohlenwasserstoffe, z. B. Toluol, chlorierte Kohlenwasserstoffe, z. B. Chlorbenzol, oder Ether, wie z. B. Dioxan.

Das erfindungsgemäße Verfahren wird ohne Zusatz eines säurebindenden Mittels durchgeführt. Zweckmäßigerweise wird Chlorcarbonylisocyanat in Lösung vorgelegt und soll im Überschuß im Reaktionsgemisch vorhanden sein. Das Molverhältnis von Chlorcarbonylisocyanat zu Sulfenamid kann in einem Bereich von 1 : 1 bis 10 : 1, vorzugsweise von 1,1 : 1 bis 2 : 1 variieren.

Die Reaktionstemperaturen können in einem größeren Bereich variiert werden. Im allgemeinen arbeitet man, wie oben angegeben, zwischen 0 und 150 °C, vorzugsweise bei + 20 bis 120 °C.

Die Isolierung der erfindungsgemäßen Verbindungen erfolgt in einfacher Weise durch Destillation.

Die erfindungsgemäßen neuen N-(Sulfenamido)-acylisocyanate der Formel I können als Zwischenprodukte zur Herstellung neuer N-sulfenylierter Biurete verwendet werden, indem man primäre oder sekundäre Amine im Temperaturbereich von etwa + 20 bis 50 °C addiert (vgl. Deutsche Patentanmeldung P vom [Le A 20 708]). Am interessanten Folgeprodukten seien z. B. die Verbindungen der Formel

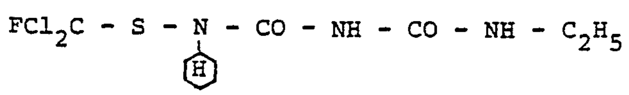

und

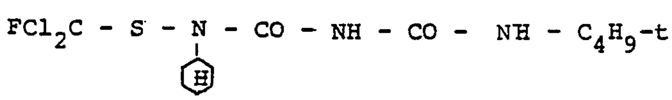

aufgezeigt (vgl. auch die Herstellungbeispiele).

Diese Verbindungen können als Pflanzenschutzmittel verwendet werden ; sie besitzen eine sehr gute fungizide Wirkung gegen phytopathogene Pilze. So können sie z. B. mit Erfolg zur Bekämpfung von Pyricularia oryzae und Pellicularia sasakii in Reiskulturen Verwendung finden und übertreffen die Wirkung bekannter Handelsprodukte.

Herstellungbeispiele

Beispiel 1

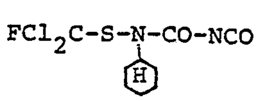

In die Lösung von 44 g (0,4 Mol) Chlorcarbonylisocyanat in 150 ml Chlorbenzol tropft man unter Eiskühlung bei + 10 bis 20 °C eine Lösung von 45 g (0,2 Mol) Fluordichlormethan-sulfenyl-N-(cyclohexyl)-amid ($Kp._{0,1}$ 68 bis 70 °C) in 50 ml Chlorbenzol ein. Anschließend erhitzt man die Reaktionslösung allmählich zum Sieden, wobei ab etwa 70 bis 80 °C Chlorwasserstoff abgespalten wird. Man hält 1 Stunde bei Rückflußtemperatur, engt die Lösung im Vakuum ein und destilliert den Rückstand. Man erhält 46 g N-(Fluordichlormethan-sulfenyl)-N-(cyclohexyl)-amido-carbonylisocyanat vom $Kp._{11}$ 158 bis 160 °C. Die Ausbeute beträgt 76 % der Theorie.

Beispiel 2

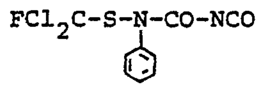

In die Lösung von 105 g (1 Mol) Chlorcarbonylisocyanat in 200 ml Chlorbenzol tropft man bei + 5 bis 10 °C 185 g (0,82 Mol) Fluordichlormethan-sulfen-anilid in 100 ml Chlorbenzol ein. Unter Rotfärbung der Lösung tritt eine Nachreaktion ein, die Temperatur steigt bis 44 °C an. Anschließend heizt man die Lösung auf ; ab etwa 80 °C setzt unter Kristallisation Chlorwasserstoffent-wicklung ein. Man erhitzt 1 Stunde lang zum Sieden, filtriert vom Festprodukt (ca. 48 g) ab, engt die Lösung im Vakuum ein und destilliert. Man erhält 25 g N-(Fluordichlormethan-sulfenyl)-anilido-carbonylisocyanat vom $Kp._{0,2}$ 122 bis 125 °C. Die Ausbeute beträgt 11 % der Theorie.

In ähnlicher Weise werden die folgenden Verbindungen der allgemeinen Formel

$$R^1 - S - \underset{R^2}{N} - CO - NCO \qquad (I)$$

erhalten :

| Beispiel Nr. | $R^1$ | $R^2$ | Kp (mm Hg / °C) |
|---|---|---|---|
| 3 | $CFCl_2$ | $CH_3$ | 14 / 93 - 96 |
| 4 | $CFCl_2$ | $CH_2-CH_2-OCH_3$ | 0,1 / 78 - 80 |
| 5 | $CCl_3$ | $C_4H_9$ - n | 0,1 / 90 - 92 |

Vorprodukte

Beispiel V1

$FCl_2C—S—NH—C(CH_3)_3$

73 g (1 Mol) tert. Butylamin werden in 300 ml Toluol gelöst und unter Kühlung bei 20 bis 30 °C tropfenweise mit 85 g (1/2 Mol) Fluordichlormethan-sulfenylchlorid versetzt. Man schüttelt die Lösung mit Wasser aus, trocknet über Natriumsulfat, engt die Toluol-Lösung im Vakuum ein und destilliert den Rückstand. Man erhält 80 g (das sind 77 % der Theorie) Flurdichlormethan-sulfenyl-N-(t-butyl)-amid vom $Kp._{13}$ 60 bis 65 °C.

Folgeprodukte

Beispiel F1

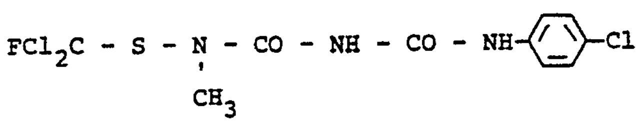

8,2 g (0,035 Mol) N-(Fluordichlormethansulfenyl)-N-(methyl)-amidocarbonylisocyanat werden in 60 ml Aceton gelöst und bei Raumtemperatur mit einer Lösung von 4,5 g (0,035 Mol) 4-Chloranilin in 20 ml Aceton umgesetzt. Hierbei steigt die Temperatur bis 34 °C an. Auf Zusatz von Wasser erhält man 7 g

1-Fluordichlormethylthio-1-methyl-5-(4-chlorphenyl)-biuret vom Fp. 148 °C, das sind 55 % der Theorie.

**Ansprüche**

1. N-(Sulfenamido)-acylisocyanate der Formel

$$R^1-S-\underset{\displaystyle R^2}{\underset{|}{N}}-CO-NCO \qquad (I)$$

in welcher
$R^1$ für einen Trihalogenmethyl-Rest steht und
$R^2$ für einen aliphatischen, cycloaliphatischen, araliphatischen, aromatischen oder heterocyclischen Rest steht.

2. Verbindungen der Formel I in Anspruch 1, in welcher
$R^1$ für einen Trichlormethyl-, Fluordichlormethyl-, Difluorchlormethyl- oder Trifluormethyl-Rest steht, und
$R^2$ für einen geradkettigen oder verzweigten Alkylrest mit 1 bis 18 Kohlenstoffatomen steht, der gegebenenfalls durch Alkoxy- und Alkylmercapto-Gruppen mit jeweils bis zu 4 Kohlenstoffatomen, ferner durch Halogen, Cyano- und Nitro-Gruppen substituiert sein kann, sodenn für einen Alkenyl- oder Alkinyl-Rest mit jeweils 3 bis 8 Kohlenstoffatomen, und für einen cycloaliphatischen Rest mit 5 bis 8 Kohlenstoffatomen steht, wobei letzterer gegebenenfalls durch einen Alkylrest mit 1 bis 4 Kohlenstoffatomen substituiert sein kann, ferner für einen araliphatischen Rest mit insgesamt 7 bis 12 Kohlenstoffatomen steht, dessen aromatisches Ringsystem gegebenenfalls durch Halogen, Nitro-Trifluormethyl-, Cyano-, Alkyl- und Alkoxy-Gruppen mit jeweils bis zu 4 Kohlenstoffatomen substituiert sein kann, weiterhin noch für einen aromatischen Rest mit 6 bis 12 Kohlenstoffatomen steht, der gegebenenfalls durch Halogen, Nitro-, Halogenalkyl-, Alkyl-, Alkoxy-, Halogenalkoxy-, Alkylthio- und Halogen-alkylthio- mit jeweils bis zu 4 Kohlenstoffatomen und Cyano-Gruppen substituiert sein kann, und endlich für einen heterocyclischen Rest mit 5 bis 6 Ringatomen steht, wobei 1 bis 3 Heteroatome wie Sauerstoff, Schwefel und/oder Stickstoff im Ringsystem vorhanden sein können.

3. Verbindungen der Formel I in Anspruch 1, in welcher
$R^1$ für einen Trichlormethyl- oder Fluordichlormethyl-Rest steht und
$R^2$ für Alkyl mit bis zu 6 Kohlenstoffatomen steht, welches substituiert sein kann durch Halogen, Methoxy-, Ethoxy- und Methylthio-Gruppen, ferner für Cyclohexyl und für Phenyl steht, welches substituiert sein kann durch Halogen, Nitro-, Methyl-, Trifluormethyl- und Methoxy-Gruppen.

4. Verfahren zur Herstellung von N-(Sulfenamido)-acylisocyanaten der Formel

$$R^1-S-\underset{\displaystyle R^2}{\underset{|}{N}}-CO-NCO \qquad (I)$$

in welcher
$R^1$ für einen Trihalogenmethyl-Rest steht und
$R^2$ für einen aliphatischen, cycloaliphatischen, araliphatischen, aromatischen oder heterocyclischen Rest steht,
dadurch gekennzeichnet, daß man Trihalogenmethan-sulfenamide der Formel

$$R^1-S-\underset{\displaystyle R^2}{\underset{|}{NH}} \qquad (II)$$

in welcher
$R^1$ und $R^2$ die oben angegebene Bedeutung besitzen,
mit Chlorcarbonylisocyanat der Formel

$$Cl—CO—NCO \qquad (III)$$

gegebenenfalls in Gegenwart eines Verdünnungsmittels im Temperaturbereich zwischen 0 und 150 °C umsetzt.

5. Verwendung von N-(Sulfenamido)-acylisocyanaten der Formel I in Ansprüchen 1 und 3 als Zwischenprodukte zur Herstellung von Pflanzenschutzmitteln mit fungizider Wirkung.

## Claims

1. N-(Sulphenamido)-acyl isocyanates of the formula

$$R^1 - S - \underset{\substack{| \\ R^2}}{N} - CO - NCO \qquad (I)$$

in which
$R^1$ represents a trihalogenomethyl radical and
$R^2$ represents an aliphatic, cycloaliphatic, araliphatic, aromatic or heterocyclic radical.

2. Compounds of the formula I in Claim 1, in which
$R^1$ represents a trichloromethyl, fluorodichloromethyl, difluorochloromethyl or trifluoromethyl radical and
$R^2$ represents a straight-chain or branched alkyl radical with 1 to 18 carbon atoms, which can optionally be substituted by alkoxy and alkylmercapto groups, in each case with up to 4 carbon atoms, and also by halogen, cyano and nitro groups, or represents an alkenyl or alkinyl radical, in each case with 3 to 8 carbon atoms, or represents a cycloaliphatic radical with 5 to 8 carbon atoms, which can optionally be substituted by an alkyl radical with 1 to 4 carbon atoms, or represents an araliphatic radical with a total of 7 to 12 carbon atoms, of which the aromatic ring system can optionally be substituted by halogen, nitro, trifluoromethyl, cyano, alkyl and alkoxy groups in each case with up to 4 carbon atoms, or represents an aromatic radical with 6 to 12 carbon atoms, which can optionally be substituted by halogen, nitro, halogenalkyl, alkyl, alkoxy, halogenalkoxy, alkylthio and halogenalkylthio groups in each case with up to 4 carbon atoms, and cyano groups or, finally, represents a heterocyclic radical with 5 to 6 ring atoms, in which 1 to 3 hetero-atoms, such as oxygen, sulphur and/or nitrogen, can be present in the ring system.

3. Compounds of the formula I in Claim 1, in which
$R^1$ represents a trichloromethyl or fluorodichloromethyl radical and
$R^2$ represents alkyl with up to 6 carbon atoms, which can be substituted by halogen, methoxy, ethoxy and methylthio groups, or represents cyclohexyl, or represents phenyl which can be substituted by halogen, nitro, methyl, trifluoromethyl and methoxy groups.

4. Process for the preparation of N-(sulphenamido)-acyl isocyanates of the formula

$$R^1 - S - \underset{\substack{| \\ R^2}}{N} - CO - NCO \qquad (I)$$

in which
$R^1$ represents a trihalogenomethyl radical and
$R^2$ represents an aliphatic, cycloaliphatic, araliphatic, aromatic or heterocyclic radical, characterised in that trihalogenomethanesulphenamides of the formula

$$R^1 - S - \underset{\substack{| \\ R^2}}{NH} \qquad (II)$$

in which
$R^1$ and $R^2$ have the abovementioned meaning are reacted with chlorocarbonyl isocyanate of the formula

$$Cl—CO—NCO \qquad (III)$$

if appropriate in the presence of a diluent, in the temperature range of between 0 and 150 °C.

5. Use of N-(sulphenamido)-acyl isocyanates of the formula I in Claims 1 and 3 as intermediate products for the preparation of plant protection agents having a fungicidal action.

## Revendications

1. N-(sulfénamido)-acylisocyanates de formule générale

$$R^1 - S - \underset{\substack{| \\ R^2}}{N} - CO - NCO \qquad (I)$$

dans laquelle

$R^1$ représente un reste trihalogénométhyle, et

$R^2$ représente un reste aliphatique, cycloaliphatique, arylaliphatique, aromatique ou hétérocyclique.

2. Composés de formule I dans la revendication 1, dans laquelle

$R^1$ représente un reste trichlorométhyle, fluorodichlorométhyle, difluorochlorométhyle ou trifluorométhyle, et

$R^2$ représente un reste alkyle à chaîne droite ou ramifiée en $C_1$-$C_{18}$, qui peut éventuellement être substitué par des groupes alcoxy- et alkylmercapto, chacun jusqu'en $C_4$, en outre par des halogènes, des groupes cyano et des groupes nitro, en outre un reste alcényle ou alcynyle, chacun en $C_3$-$C_8$, et un reste cycloaliphatique en $C_5$-$C_8$, ce dernier pouvant éventuellement être substitué par un reste alkyle en $C_1$-$C_4$, en outre un reste arylaliphatique en $C_7$-$C_{12}$ au total, dont le système cyclique aromatique peut éventuellement être substitué par des halogènes ou des groupes nitro, trifluorométhyle, cyano, alkyle et alcoxy, chacun jusqu'en $C_4$, en outre encore un reste aromatique en $C_6$-$C_{12}$, qui peut éventuellement être substitué par des halogènes ou des groupes nitro, halogénoalkyle, alkyle, alcoxy, halogénoalcoxy, alkylthio et halogénoalkythio, chacun jusqu'en $C_4$, et des groupes cyano et enfin un reste hétérocyclique ayant 5 à 6 atomes cycliques, le système cyclique pouvant contenir 1 à 3 hétéroatomes tels qu'oxygène, soufre et/ou azote.

3. Composés de formule I dans la revendication 1, dans laquelle

$R^1$ représente un reste trichlorométhyle ou fluorodichlorométhyle, et

$R^2$ représente un groupe alkyle jusqu'en $C_6$, qui peut être substitué par des halogènes et des groupes méthoxy, éthoxy et méthylthio, en outre un reste cyclohexyle et un reste phényle, qui peut être substitué par des halogènes et des groupes nitro, méthyle, trifluorométhyle et méthoxy.

4. Procédé pour la fabrication de N-(sulfénamido)-acylisocyanates de formule

$$R^1 - S - \underset{\displaystyle R^2}{\underset{|}{N}} - CO - NCO \qquad (I)$$

dans laquelle

$R^1$ représente un reste trihalogénométhyle, et

$R^2$ représente un reste aliphatique, cycloaliphatique, arylaliphatique, aromatique ou hétérocyclique, caractérisé en ce que l'on fait réagir des trihalogénométhanesulfénamides de formule générale

$$R^1 - S - \underset{\displaystyle R^2}{\underset{|}{NH}} \qquad (II)$$

dans laquelle

$R^1$ et $R^2$ ont la signification indiquée ci-dessus, avec l'isocyanate de chlorocarbonyle de formule

$$Cl—CO—NCO \qquad (III)$$

dans l'intervalle de températures de 0 à 150 °C, éventuellement en présence d'un diluant.

5. Utilisation des N-(sulfénamido)-acylisocyanates de formule (I) dans les revendications 1 et 3 comme produits intermédiaires pour la fabrication d'agents phytosanitaires doués d'activité fongicide.